# EUROPEAN PATENT SPECIFICATION

(11) **EP 0 551 953 B1**
(45) Date of publication and mention of the grant of the patent: **05.06.1996**
(21) Application number: 93200087.0
(22) Date of filing: 14.01.1993
(51) Int. Cl.: C07J 43/00, A61K 31/58

(54) **17-(Pyridazine-N-oxide)-substituted steroidal compounds active on the cardiovascular system**
17-(Pyridazin-N-Oxyd)-substituierte Steroide mit cardiotonischer Wirksamkeit
Dérivés 17-(pyridazine-N-oxyde)-substitués des steroides, avec activité sur le système cardiovasculaire

(30) Priority: 16.01.1992 IT MI920075
(43) Date of publication of application: 21.07.1993
(73) Proprietor: ZAMBON GROUP S.p.A., I-36100 Vicenza (IT)
(72) Inventor: Bertolini, Giorgio, I-20099 Sesto S. Giovanni (Milan) (IT); Casagrande, Cesare, I-20020 Arese (Milan) (IT); Norcini, Gabriele, I-21019 Maddalena Somma Lombardo VA (IT); Santangelo, Francesco, I-20148 Milan (IT)
(74) Representative: Marchi, Massimo

(56) References cited:
- FR-A- 2 281 124
- STEROIDS. vol. 42, no. 2 , 1983 , SAN FRANCISCO US pages 189 - 203 Humber D C et al 'Synthesis and biological activity of some cardiotonic compounds related to digitoxigenin'
- EUROPEAN JOURNAL OF MEDICINAL CHEMISTRY.CHIMICA THERAPEUTICA vol. 21, no. 3 , June 1986 , PARIS FR pages 193 - 198 Bohl M et al 'Calculations on molecular structure and electrostatic potentials of cardiotonic steroids'
- DRUGS EXP. CLIN. RES. vol. 9, no. 3 , 1983 pages 207 - 212 Marzo A et al 'The pharmacokinetic behavior of SC4453, a new semisynthetic cardiac glycoside, and digoxin in the dog'
- J. PHARMACOL. vol. 15, no. 1 , 1984 pages 53 - 64 F. RANDIMBIVOLOLONA ET AL 'Metabolism and excretion in bile of SC4453, a new semisynthetic derivative of digoxin following an i.v. bolus injection in the guinea pig'

## Description

The present invention relates to steroidal compounds active on the cardiovascular system.

More particularly, it relates to cardioactive steroidal compounds substituted with a pyridazine-N-oxide nucleus at the 17-position.

Naturally occurring steroidal compounds endowed with a digitalis-like cardiotonic activity have an unsaturated lactone ring at the 17-position.

Studies on molecular modifications have shown that the natural lactone ring can be replaced by other groups while maintaining, to a certain degree, the digitalis-like activity (see, for example, K. R. H. Repke, TIPS - July 1985 - Elsevier Science Publishers B. V. Amsterdam, pp. 275-278).

Among the most important examples, described in the literature, of groups replacing the natural ring there are heterocyclic structures such as lactone isomers, furans, pyridine, pyridazine or open structures.

In particular, the compounds substituted with a pyridazine nucleus at the 17-position which are described in the US patent 4,000,125 (Siphar S.A.) have become especially interesting since, apart from generally possessing a better pharmaceutical activity compared to the corresponding naturally occurring molecules, they exhibit a better therapeutic index, or rather a lower toxicity.

Among the modifications of the compounds described in the aforementioned US patent, the oxidation of the pyridazine ring has already been studied and disclosed (Humber et al., Steroids, 42(2), pp. 189-203, (1983)).

However, the above cited work states that the said oxidation, when performed on compounds of the formula 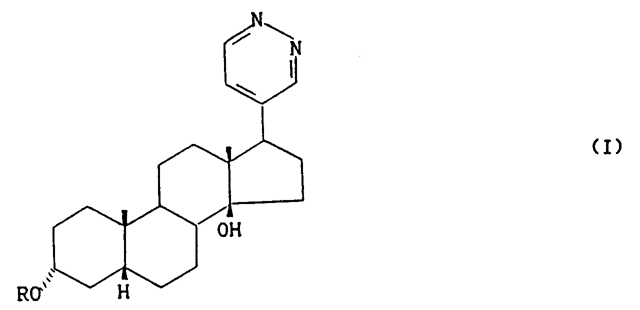
where R is hydrogen or acetyl;
does not improve the pharmaceutical activity with respect to the corresponding naturally occurring molecule, digitoxygenin, or with respect to the pyridazine starting compound I (Table 1, page 194 of the above cited article by Humber et al.).

In addition, it is known that the pharmacological activity of the cardioactive steroidal compounds (glycosides) resides in that portion of the molecule which is commonly called aglycone or genin, i.e. the portion without the glycosyl group (Brian F. Hoffman and J. Thomas Bigger Jr. - Goodman and Gilman's The Pharmacological Basis of Therapeutics VII ed. - Chapter 30, pp. 716-747).

Generally, the glycosyl group improves the pharmacokinetic properties of the compound.

Now, we have surprisingly found that introduction of a glycosyl group at position 3 of the 17-(pyridazine-N-oxide)-substituted genins yields compounds which not only exhibit a notably higher pharmacological activity compared to the corresponding 17-pyridazine-substituted compounds, but which are moreover characterized by having a high selectivity for the high affinity receptors, such as the alpha₃ and alpha₂ isoforms of the enzyme (Na⁺+K⁺)-ATPase.

It is therefore an object of this invention to provide the compounds of the formula 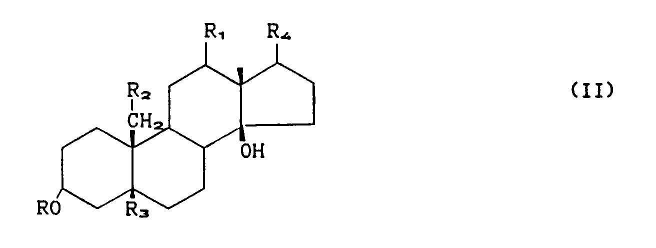
wherein
- R: is a glycosyl group;
- R₁ and R₂: are hydrogen or hydroxy groups optionally esterified by a carboxylic acid selected among formic, acetic, propionic and butyric acid;
- R₃: is hydrogen or hydroxy;
- R₄: is a 4-pyridazyl-1-N-oxide or 4-pyridazyl-2-N-oxide group;
and the substituents at the positions 5, 10, 13 and 14 have beta configuration.

The compounds of formula II of this invention, are useful in the therapy of pathologies of the cardiovascular system, particularly in the treatment of heart failure and hypertension, owing to their improved therapeutic index and lack of nonspecific and toxic effects depending on the binding to low affinity receptors such as the alpha₁ isoforms of the (Na⁺+K⁺)-ATPase.

Specific examples of glycosyl groups included in the meaning of R are the monosaccharides such as D-glucose, Lyxsose, D-xylose, 2-deoxy-D-glucose, D-allomethylose, D-thevetose, L-thevetose, L-talomethylose, D-gulomethylose, D-glucomethylose, L-rhamnose, L-acofriose, D-fucose, L-fucose, D-digitalose, L-acovenose, D-digitoxose, D-cymarose, D-boivinose, D-sarmentose, L-oleandrose, D-oleandrose, D-diginose, as well as the di- and trisaccharides consisting, respectively, of two and three of the above mentioned monosaccharides.

Particularly preferred compounds of formula II are those wherein R is a monosaccharide selected from L-rhamnose and L-thevetose, or a trisaccharide, consisting of three molecules of D-digitoxose.

The preparation of the compounds of Formula II is carried out by oxidation of the corresponding 17-pyridazine-substituted compound of the following formula 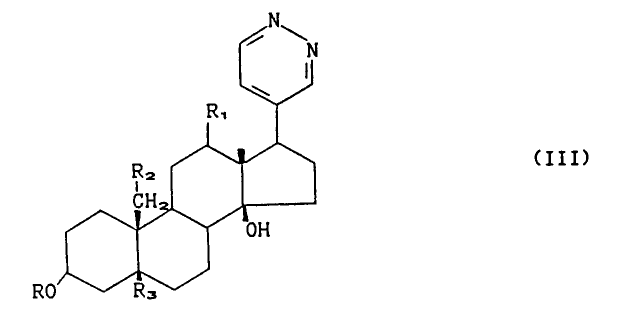
where R, R₁, R₂ and R₃ are as described above.

The oxidation reaction is performed according to known techniques, consistent with the functional groups present in the molecule. For example, the oxidation can be carried out using peracids such as peracetic acid, perbenzoic acid, m.chloroperbenzoic acid or monoperoxyphthalic acid or hydroperoxides such as t.pentylhydroperoxide in the presence of molybdenum pentachloride.

Generally, the oxidation reaction produces a mixture of compounds of formula II 17-(4-pyridazil-1-N-oxide) and 17-(4-pyridazil-2-N-oxide) substituted, which can be separated according to conventional techniques such as chromatography and fractional crystallization.

The compounds of formula III are well known and have been disclosed in the aforementioned US patent 4,000,125.

The compounds of formula II according to this invention, are useful in the therapy of pathologies of the cardiovascular system since they possess positive inotropic activity, sometimes associated with a vasodilating activity, and thereby they exercise favourable effects, especially in the heart failure and hypertension.

The activity of the compounds of formula II has been evaluated with the binding test on the alpha₁ and alpha₃ isoforms of rat (Na⁺+K⁺)-ATPase in rat kidney and rat brain, according to the method described by Noel F. et al., Biochemical Pharmacology, 40(12), pp 2611-2616, (1990) (see Example 2).

Practically, the compounds of formula II do not bind themselves to the alpha₁ isoforms while they interact at very low concentrations with the alpha₃ isoforms (see Example 2).

As a consequence, their therapeutic index (expressed as a ratio between the K₁ of the alpha₁ and alpha₃ isoforms) is at least 20-50 times higher compared to the naturally occurring cardioactive glycosides and higher than that of the corresponding 17-pyridazine-substituted derivatives.

Another particularly important feature of the compounds of formula II is that their pharmacological activity is surprisingly higher than that of the corresponding genins.

In particular, in the binding test on the alpha₁ and alpha₃ isoforms of rat (Na⁺+K⁺)-ATPase, the genins of formula I (17-pyridazine-substituted) exhibited a slightly higher activity with respect to the corresponding naturally occurring genins while the analogous N-oxides were practically inactive (see Example 2).

For practical therapeutic applications, the compounds of formula II are formulated into pharmaceutically acceptable forms suitable for oral or parenteral application.

In general, the therapeutic doses are of from 0.01 to 100 mg/day.

It is therefore a further object of the present invention to provide pharmaceutical compositions containing a therapeutically effective amount of a compound of formula II together with a pharmaceutical carrier.

The compositions of the present invention are prepared according to conventional techniques.

The following examples are given to further illustrate the present invention.

### Example 1

### Preparation of 3beta-(tris-beta-D-digitoxopyranosyloxy)-12beta,14-dihydroxy-17beta-(4-pyridazinyl-2-N-oxide)-5beta,14beta-androstane (Compound 1) and 3beta-(tris-beta-D-digitoxopyranosyloxy)-12beta,14-di-hydroxy-17beta-(4-pyridazinyl-1-N-oxide)-5beta,14beta-androstane (Compound 2)

To a solution of 3beta-(tris-beta-D-digitoxopyranosyloxy)-12beta,14-dihydroxy-17beta-(4-pyridazyl)-5beta,14beta-androstane (10 g; 12.87 mmoles), in methylene chlorine (4 ml), m.chloroperbenzoic acid (7.8 g; 38.61 mmoles) is added, and the reaction mixture is kept under stirring overnight.

The resulting solution is then concentrated to small volume (approximately 100 ml) and diluted with ethyl ether (100 ml).

The precipitate is then washed with ethyl ether (2 x 50 ml).

It is thus obtained a mixture of Compounds 1 and 2 which are separated and purified by column chromatography (silica gel, methylene chloride : methyl alcohol = 9 : 1).

The two separated compounds are then suspended in ethyl acetate.

The suspension is then refluxed to obtain, after filtration and drying:
Compound 1 (1.1 g), m.p. 248-249°C (dec)
¹H-NMR (300 MHz, DMSO-d₆): delta (ppm): 0.43 (s, 3H); 0.84 (s, 3H); 1.09 (d, 3H); 1.11 (d, 3H); 1.12 (d, 3H); 1.00-2.00 (m, 24H); 2.13 (m, 1H); 3.00 (m, 1H); 3.12 (d, 2H); 3.27 (m, 2H); 3.68 (m, 3H); 3.86 (bt, 1H); 3.91 (bs, 1H); 4.04 (m, 2H); 4.78 (d, 1H); 4.81 (d, 2H); 7.18 (d, 1H); 8.22 (s, 1H); 8.42 (d, 1H).
Compound 2 (1.0 g), m.p. 239-240°C (dec)
¹H-NMR (300 MHz, DMSO-d₆): delta (ppm): 0.41 (s, 3H); 0.85 (s, 3H); 1.10 (d, 3H); 1.12 (d, 3H); 1.13 (d, 3H); 1.00-2.00 (m, 24H); 2.16 (m, 1H); 3.00 (m, 1H); 3.13 (d, 2H); 3.30 (m, 2H); 3.67 (m, 3H); 3.84 (bt, 1H); 3.91 (bs, 1H); 4.05 (m, 2H); 4.77 (d, 1H); 4.81 (d, 2H); 7.75 (dd, 1H); 8.22 (d, 1H); 8.38 (d, 1H).

The following compounds were prepared in a similar way

### 3beta-(tris-beta-D-digitoxopyranosyloxy)-14-hydroxy-17beta-(4-pyridazinyl-1-N-oxide)-5beta,14beta-androstane (Compound 3)

m.p. 233-235°C (dec)
¹H-NMR (300 MHz, DMSO-d₆): delta (ppm): 0.52 (s, 3H); 0.86 (s, 3H); 1.09 (d, 3H); 1.11 (d, 3H); 1.12 (d, 3H); 1.00-1.90 (m, 25H); 2.16 (m, 2H); 2.80 (dd, 1H); 3.00 (m, 1H); 3.12 (d, 2H); 3.68 (m, 3H); 3.86 (bt, 1H); 3.92 (bs, 1H); 4.06 (m, 2H); 4.78 (dd, 1H); 4.81 (dd, 2H); 7.86 (dd, 1H); 8.22 (d, 1H); 8.47 (d, 1H).

### 3beta-(tris-beta-D-digitoxopyranosyloxy)-14-hydroxy-17beta-(4-pyridazinyl-2-N-oxide)-5beta,14beta-androstane (Compound 4)

m.p. 269-272°C (dec)
¹H-NMR (300 MHz, DMSO-d₆): delta (ppm): 0.56 (s, 3H); 0.85 (s, 3H); 1.09 (d, 3H); 1.11 (d, 3H); 1.12 (d, 3H); 1.00-1.90 (m, 25H); 2.16 (m, 2H); 2.78 (dd, 1H); 3.00 (m, 1H); 3.13 (d, 2H); 3.68 (m, 3H); 3.86 (bt, 1H); 3.91 (bs, 1H); 4.06 (m, 2H); 4.78 (dd, 1H); 4.81 (dd, 2H); 7.30 (d, 1H); 8.36 (s, 1H); 8.41 (d, 1H).

### 3beta-(alfa-L-thevetopyranosyloxy)-14-hydroxy-17beta-(4-pyridazinyl-2-N-oxide)-5beta,14beta-androstane (Compound 5)

m.p. 249-251°C
¹H-NMR (300 MHz, CDCl₃): delta (ppm): 0.67 (s, 3H); 0.95 (s, 3H); 1.25 (d, 3H); 1.10-2.40 (m, 20H); 2.71 (dd, 1H); 3.15 (t, 1H); 3.25 (t, 1H); 3.58 (dd, 1H); 3.68 (s, 3H); 3.74 (dd, 1H); 3.97 (m, 1H); 4.85 (d, 1H); 7.18 (d, 1H); 8.27 (s, 1H); 8.29 (d, 1H).

### 3beta-(alfa-L-thevetopyranosyloxy)-14-hydroxy-17beta-(4-pyridazinyl-1-N-oxide)-5beta,14beta-androstane (Compound 6)

m.p. 270-272°C
¹H-NMR (300 MHz, CDCl₃): delta (ppm): 0.63 (s, 3H); 0.95 (s, 3H); 1.26 (d, 3H); 1.10-2.50 (m, 20H); 2.80 (dd, 1H); 3.15 (dd, 1H); 3.25 (dd, 1H); 3.59 (m, 1H); 3.68 (s, 3H); 3.75 (dd, 1H); 3.97 (bs, 1H); 4.86 (d, 1H); 7.78 (dd, 1H); 8.06 (d, 1H); 8.37 (d, 1H).

### Example 2

### Evaluation of the interaction with the alpha₁ and alpha₃ isoforms of rat (Na⁺+K⁺)-ATPase

The method of Noel F. et al., Biochemical Pharmacology, 40(12), pp. 2611-2616, (1990) was used to evaluate the displacement of [³H]-ouabain in preparations of rat kidney and rat brain, containing various concentrations of neriifolin, 3beta-(alpha-L-thevetopyranosyloxy)-14-hydroxy-17beta-(4-pyridazinyl)-5beta,14beta-androstane (compound R), compound 5 and compound 6 and the corresponding genins, respectively, digitoxygenin, 3beta,14-dihydroxy-17beta-(4-pyridazinyl)-5beta,14beta-androstane (genin 1), 3beta,14-dihydroxy-17beta-(4-pyridazinyl-2-N-oxide)-5beta,14beta-androstane (genin 2) and 3beta,14-dihydroxy-17beta-(4-pyridazinyl-1-N-oxide)-5beta,14beta-androstane (genin 3).

The results, expressed as K₁(mmoles) and as a selectivity index (that is, K₁(alpha₁)/K₁(alpha₃)), are reported in the following table.

**Table 1**

| Displacement of [³H]-ouabain expressed as K₁(mM) in preparations of rat kidney and rat brain in the presence of neriifolin, compound R, compound 5, compound 6, digitoxygenin, genin 1, genin 2 and genin 3. | | | |
|---|---|---|---|
| | alpha₁ isoform K₁ (mM) | alpha₃ isoform K₁ (mM) | K₁(alpha₁)/K₁(alpha₃) |
| Nerifolin | 4.4 | 0.049 | 90 |
| Compound R | 2.7 | 0.043 | 63 |
| Compound 5 | ≧100.0 | 0.08 | ≧1258 |
| Compound 6 | ≧100.0 | 0.12 | ≧1000 |
| Digitoxygenin | 19.0 | 0.52 | 37 |
| Genin 1 | 25.0 | 0.26 | 96 |
| Genin 2 | >10.0 | >10.0 | -- |
| Genin 3 | >10.0 | >10.0 | -- |

These data show that the compounds of this invention selectively interact with high affinity sites (alpha₃ isoform) significantly differentiating themselves from the corresponding naturally occurring compounds and from the analogous compounds which are not oxidized on the pyridazine nucleus and, above all, from the corresponding genins.

## Claims

1. A compound of the formula 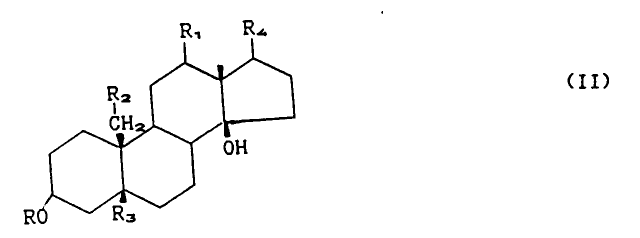 wherein
R is is a glycosyl group;
R₁ and R₂ are hydrogen or hydroxy groups optionally esterified by a carboxylic acid selected among formic, acetic, propionic and butyric acid;
R₃ is hydrogen or hydroxy;
R₄ is a 4-pyridazinyl-1-N-oxide or 4-pyridazinyl-2-N-oxide group; and the substituents at the positions 5, 10, 13 and 14 have beta configuration.

2. A compound according to claim 1 wherein R is a monosaccharide selected from L-rhamnose and L-thevetose or a trisaccharide, consisting of three molecules of D-digitoxose.

3. A pharmaceutical composition containing a therapeutically effective amount of a compound according to claim 1 together with a pharmaceutical carrier.

## Patentansprüche

1. Verbindung der Formel 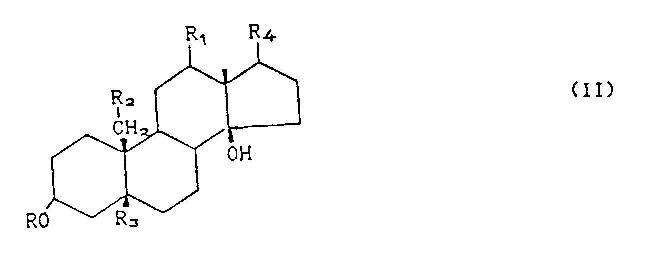 worin
R für eine Glycosylgruppe steht;
R₁ und R₂ für Wasserstoffatome oder Hydroxylgruppen stehen, die gegebenenfalls mit einer Carbonsäure verestert sind, welche unter Ameisen-, Essig-, Propion- und Buttersäure ausgewählt ist;
R₃ für ein Wasserstoffatom oder eine Hydroxylgruppe steht;
R₄ für eine 4-Pyridazinyl-1-N-oxid- oder 4-Pyridazinyl-2-N-oxid-Gruppe steht;
und die Substituenten an den Positionen 5, 10, 13 und 14 beta-Konfiguration aufweisen.

2. Verbindung nach Anspruch 1, worin R für ein Monosaccharid steht, welches unter L-Rhamnose und L-Thevetose ausgewählt ist, oder für ein Trisaccharid steht, welches aus drei Molekülen D-Digitoxose besteht.

3. Pharmazeutisches Mittel enthaltend eine therapeutisch effektive Menge einer Verbindung nach Anspruch 1 zusammen mit einem pharmazeutischen Träger.

## Revendications

1. Composé de formule 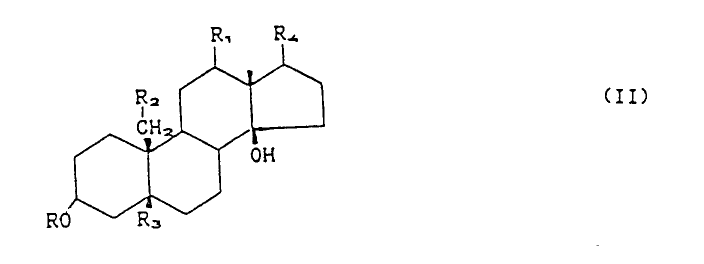 dans laquelle
R est un coupe glycosyle ;
R₁ et R₂ sont l'hydrogène ou le coupe hydroxyle éventuellement estérifié par un acide carboxylique choisi parmi les acides formique, acétique, propionique et butyrique ;
R₃ est l'hydrogène ou l'hydroxyle ;
R₄ est le coupe 4-pyridazinyl-1-N-oxyde ou 4-pyridazinyl-2-N-oxyde ; et les substituants en positions 5, 10, 13 et 14 sont en configuration béta.

2. Composé selon la revendication 1, dans lequel R est un monosaccharide choisi parmi le L-rhamnose et le L-thévétose ou un trisaccharide, composé de trois molécules de D-digitoxose.

3. Composition pharmaceutique contenant une quantité thérapeutiquement efficace d'un composé selon la revendication 1 avec un support pharmaceutique.
